# EUROPEAN PATENT SPECIFICATION

(11) **EP 4 003 394 B1**
(45) Date of publication and mention of the grant of the patent: **16.04.2025**
(21) Application number: 20756777.7
(22) Date of filing: 28.07.2020
(51) Int. Cl.: A61K 38/10, A61K 38/17, A61P 19/02, A61P 19/00, A61P 25/28, A61P 3/00, A61P 9/00, A61P 11/00, A61P 27/00, A61P 35/00

(54) **PEPTIDES FOR USE AS SENOTHERAPEUTIC AGENTS**
PEPTIDE ZUR VERWENDUNG ALS SENOTHERAPEUTIKA
PEPTIDES DESTINÉS À ÊTRE UTILISÉS EN TANT QU'AGENTS SÉNOTHÉRAPEUTIQUES

(30) Priority: 30.07.2019 ES 201930708
(43) Date of publication of application: 01.06.2022
(73) Proprietor: Fundación Profesor Novoa Santos, 15006 A Coruna (ES); Universidade de Santiago de Compostela, 15782 Santiago de Compostela (ES); Universidade Da Coruña, 15001 A Coruña (ES); Servizo Galego de Saude, 15703 Santiago de Compostela (ES)
(72) Inventor: MAYÁN SANTOS, María Dolores, 15006 A Coruña (ES); VARELA EIRÍN, Marta, 15006 A Coruña (ES); CASTRO IGLESIAS, Alejandro, 15001 A Coruña (ES); VÁZQUEZ SENTÍS, Eugenio, 15782 Santiago de Compostela (ES); MASCAREÑAS CID, José Luis, 15782 Santiago de Compostela (ES); LEARTE AYMAMÍ, Soraya, 15782 Santiago de Compostela (ES); CAEIRO REY, José Ramón, 15703 Santiago de Compostela (ES); PAZOS CHANTRERO, Elena, 15001 A Coruña (ES)
(74) Representative: Hoffmann Eitle
(86) International application number: PCT/EP2020/071242
(87) International publication number: WO 2021/018879

(56) References cited:
- WO-A1-2013/163423
- WO-A1-2017/180896
- E GANGOSO ET AL: "A cell-penetrating peptide based on the interaction between c-Src and connexin43 reverses glioma stem cell phenotype", CELL DEATH & DISEASE, vol. 5, no. 1, 1 January 2014 (2014-01-01), pages e1023 - e1023, XP055745284, DOI: 10.1038/cddis.2013.560
- LAURA GARCÍA VICENTE: "Capacidad de internalización de péptidos penetrantes en células de glioma, neuronas y astrocitos", 1 January 2016 (2016-01-01), XP055745314, Retrieved from the Internet <URL:https://gredos.usal.es/bitstream/handle/10366/135823/16TFG382_GARCÍA%20VICENTE_LAURA.pdf?sequence=1&isAllowed=y> [retrieved on 20201029]
- MARTA VARELA-EIRÍN ET AL: "Targeting of chondrocyte plasticity via connexin43 modulation attenuates cellular senescence and fosters a pro-regenerative environment in osteoarthritis", CELL DEATH & DISEASE, vol. 9, no. 12, 1 December 2018 (2018-12-01), XP055744963, DOI: 10.1038/s41419-018-1225-2
- RAQUEL GAGO-FUENTES ET AL: "The C-terminal domain of connexin43 modulates cartilage structure via chondrocyte phenotypic changes", ONCOTARGET, vol. 7, no. 45, 8 November 2016 (2016-11-08), United States, pages 73055 - 73067, XP055744964, ISSN: 1949-2553, DOI: 10.18632/oncotarget.12197
- CAEIRO JR ET AL: "Regulación de la plasticidad celular y senescencia en condrocitos articulares: conexina 43 como diana terapéutica para el tratamiento de la artrosis", REVISTA DE OSTEOPOROSIS Y METABOLISMO MINERAL, 1 June 2019 (2019-06-01), pages 46 - 54, XP055744965, Retrieved from the Internet <URL:http://scielo.isciii.es/pdf/romm/v11n2/en_1889-836X-romm-11-2-0046.pdf> DOI: 10.4321/S1889-836X2019000300003
- MARTA VARELA EIRIN: "Role of connexin43 in cell plasticity and tissue degeneration in osteoarthritis", 1 January 2018 (2018-01-01), XP055745737, Retrieved from the Internet <URL:https://ruc.udc.es/dspace/handle/2183/21066> [retrieved on 20201030]
- M. VARELA-EIRÍN ET AL: "OP0223 - Targeting chondrocyte plasticity via connexin43 modulation attenuates cellular senescence in osteoarthritis", THURSDAY, 14 JUNE 2018, 1 June 2018 (2018-06-01), pages 160.2 - 160, XP055744962, DOI: 10.1136/annrheumdis-2018-eular.4663
- MARTA VARELA-EIRIN ET AL: "Cartilage regeneration and ageing: Targeting cellular plasticity in osteoarthritis", AGEING RESEARCH REVIEWS, vol. 42, 16 December 2017 (2017-12-16), NL, pages 56 - 71, XP055725498, ISSN: 1568-1637, DOI: 10.1016/j.arr.2017.12.006
- HOWARD EVANS W ET AL: "Manipulating Connexin Communication Channels: Use of Peptidomimetics and the Translational Outputs", JOURNAL OF MEMBRANE BIOLOGY, SPRINGER-VERLAG, NE, vol. 245, no. 8, 11 August 2012 (2012-08-11), pages 437 - 449, XP035116476, ISSN: 1432-1424, DOI: 10.1007/S00232-012-9488-5
- MYRIAM JARAÍZ-RODRÍGUEZ ET AL: "Conclusion", NEURO-ONCOLOGY, vol. 22, no. 4, 28 December 2019 (2019-12-28), US, pages 493 - 504, XP055745630, ISSN: 1522-8517, DOI: 10.1093/neuonc/noz243

## Description

### FIELD OF THE INVENTION

The present invention pertains to the medical field. Precisely, the present invention refers to senotherapeutic agents comprising a peptide of SEQ ID NO: 1 or SEQ ID NO: 2. In a preferred embodiment the present invention is directed to the prevention or treatment of osteoarthritis caused by the persistence and accumulation of senescent cells (SnCs).

### STATE OF THE ART

Osteoarthritis (OA) is the most common form of arthritis and it has been classified as an aging-associated disease [1, 2]. OA is a degenerative joint disorder characterised by progressive degeneration of synovial joints that lead to limited mobility and pain [3]. This disease is the major cause of disability with a major socio-economic impact, which affects an increasing number of the ageing population [4, 5]. Articular cartilage damage is the most typical sign of OA. Yet, this condition also affects the whole joint including bone, synovium, muscles, tendons and ligaments. Articular cartilage from patients with OA shows an accumulation of dedifferentiated and SnCs together with an increase in the production of pro-inflammatory cytokines such as IL-6 and IL-1β and catabolic enzymes that lead to the breakdown of cartilage extracellular matrix (ECM) and joint degeneration. The prevalence of OA is rising worldwide and the aetiology is still under study, with new insights into molecular mechanisms involved in OA progression beginning to open new possibilities to restore phenotypic stability of articular chondrocytes and to develop new approaches in order to promote cartilage repair and restore normal joint function in OA patients [6-8].

Accumulation of SnCs has been involved in the progression of different age-related diseases such as osteoarthritis (OA) [7, 9-12]. Cellular senescence consists in a cell-cycle arrest mechanism through sustained activation of the p16^{INK4A} and/or p53-p21 pathways, which is involved in different biological processes such as development and tissue repair. Senescence cells cease dividing but are metabolic active cells, secreting factors refereed as senescence-associated secretory phenotype (SASP), which include cytokines, chemokines, growth factors and proteases [13]. This secretome is very complex and its products are mainly associated with inflammation, reprogramming, dedifferentiation and proliferation of surrounding cells together with changes in the synthesis and degradation of the extracellular matrix. The accumulation of SnCs in a chronic condition negatively impact on tissue structure and function. Examples of aging-associated diseases, which have been associated with accumulation of SnCs, are (non-exhaustive list): obesity, sarcopenia, fibrotic disease, atherosclerosis, cardiovascular disease, cancer, OA, arthritis, cataracts, osteoporosis, diabetes, Alzheimer's disease, hair loss, hypertension, inflammatory disease, dementia, kidney disease, muscular atrophy, neurological disease, pulmonary disease, vertebral disc degeneration and alopecia. The incidence of all of these diseases increases exponentially with age [9].

SnCs accumulate with age and contribute to the development of age-associated diseases. As a result, different strategies have emerged lately in order to therapeutically target SnCs. The therapeutic approach that takes advantage of compounds that target SnCs (senotherapeutics) is commonly described as senotherapy. Senotherapeutic agents comprise senolytics (selectively kill SnCs) and senomorphics / senostatics (compounds that modulate SASP). The clearance of SnCs and/or targeting of pathways involved in SASP production and secretion have been reported to alleviate the symptoms or evolution of some age-related diseases, including in OA. However, there is still a clear medical need of effective senotherapeutic agents with regenerative capacity able to selectively induce death of SnCs and/or reduce their accumulation in aged tissue in order to restore regeneration and tissue normal function.

The transmembrane protein connexin43 (Cx43) has been reported to reinforce senescence and dedifferentiation in articular chondrocytes from patients with OA and to contribute to disease progression [6]. However, Cx43 is a complex channel protein implicated in multiples biological processes. The different and diverse functions of Cx43 are specifically mediated by small domains throughout the protein sequence [14, 16-18]. Actually, several compounds including different peptide compounds have been designed and tested to modulate a specific function of the protein without affecting other functions [15, 19, 20]. Pharmacological inhibitors that specifically target certain activities of Cx43 such as therapeutic peptides are emerging as promising drugs for the treatment of different disorders such as cancer or neurodegeneration by targeting protein-protein interactions [15, 20, 21].

In fact, targeted modulation protein-protein interactions by therapeutic peptides has represented a unique and potent class of pharmaceutical compounds which is providing life-saving medicines since first half of the 20^{th} century. Over 60 peptide drugs targeting different protein domains have been already approved and over 150 new peptides are in active clinical development [22-24].

The present invention refers to peptides which can be used as senotherapeutic agents to restore regeneration for the prevention or treatment of OA caused by the persistence and accumulation of dedifferentiated and senescent cells, particularly senescent chondrocyte and dedifferentiated cells in articular cartilage and synovial joints.

The following prior art documents are acknowledged:
**D1.** WO2017180896.
**D2.** WO2013163423.
**D3.** E GANGOSO ET AL, "A cell-penetrating peptide based on the interaction between c-Src and connexin43 reverses glioma stem cell phenotype", CELL DEATH & DISEASE, (20140101), vol. 5, no. 1, doi:10.1038/cddis.2013.560.
**D4.** Laura Garcia Vicente, "Capacidad de internalización de péptidos penetrantes en células de glioma, neuronas y astrocitos", (20160101), URL: https://gredos.usal.es/bitstream/handle/10366/135823/16TFG382_GARCÍA%20VICENTE_L AURA.pdf?sequence=1&isAllowed=y
**D5.** MARTA VARELA-EIRÍN ET AL, "Targeting of chondrocyte plasticity via connexin43 modulation attenuates cellular senescence and fosters a pro-regenerative environment in osteoarthritis", CELL DEATH & DISEASE, (20181201), vol. 9, no. 12, doi:10.1038/s41419-018-1225-2.

However, none of the cited prior art documents discloses the use of peptides consisting of SEQ ID NO: 1 or SEQ ID NO: 2 as a medicament, particularly as an effective and non-toxic strategy for preventing or treating osteoarthritis.

### DESCRIPTION OF THE INVENTION

### Brief description of the invention

The present invention refers to senotherapeutic agents comprising the following peptides, and their use in the prevention or treatment of OA caused by the persistence and accumulation of senescent cells:
- SEQ ID NO: 1 (YSA1): Nt-GKSDPYHATSGALSPAKD-Ct
- SEQ ID NO: 2 (YTP1): ***Nt***-GKSDPYHATTGPLSPAKD-***Ct***

In a preferred embodiment, the peptides SEQ ID NO: 1 or SEQ ID NO: 2 have been selected from a region of the protein Cx43 that is comprised between residues 232 to 266.

In a preferred embodiment the above cited peptides can comprise a tail of 8 arginines at the N-terminal domain to facilitate the internalization of the peptides within the cell, as well as other tag such as TAT or other molecules that facilitate their internalization.

OA is assayed as an illustrative example of aging-associated diseases caused by the persistence and accumulation of senescent cells. Such as it is explained below, the peptides of the invention are able to i) reduce the accumulation of chondrocyte senescent cells for example by inducing their death via apoptosis or inhibition of the SASP and/or ii) trigger the re-differentiation of chondrocytes once they have been de-differentiated.

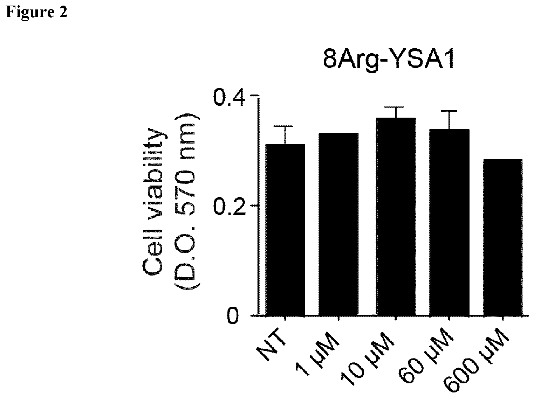

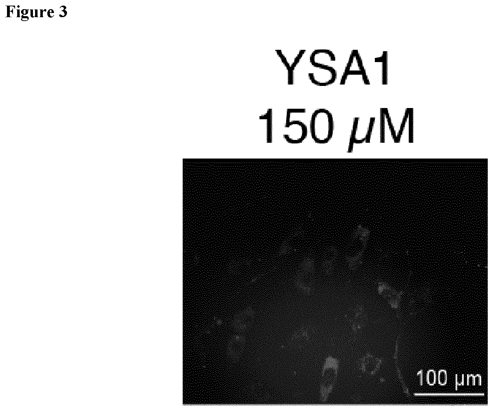

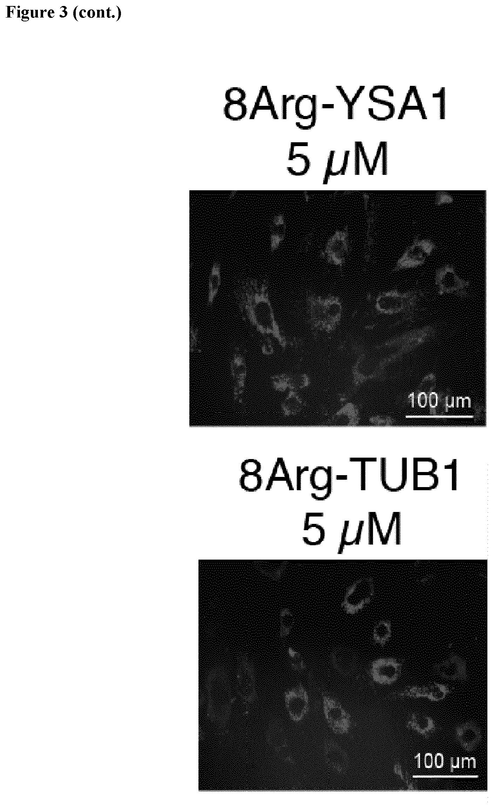

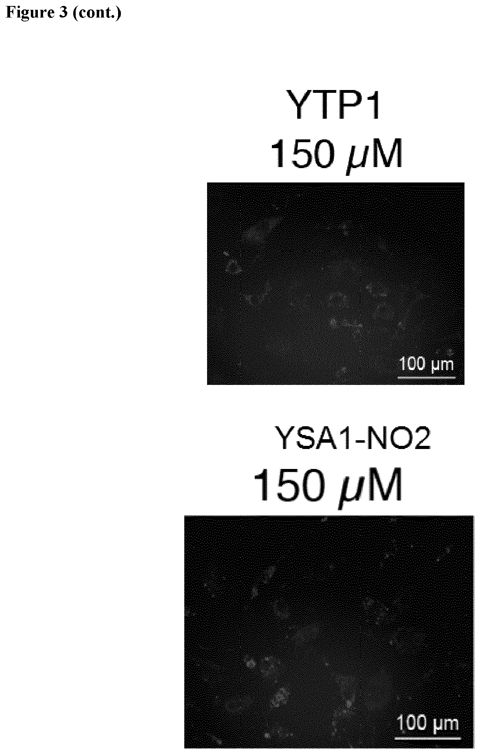

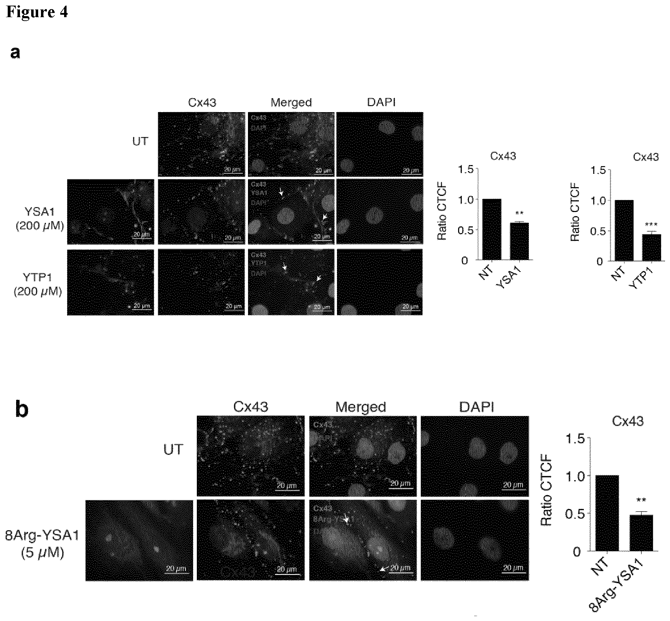

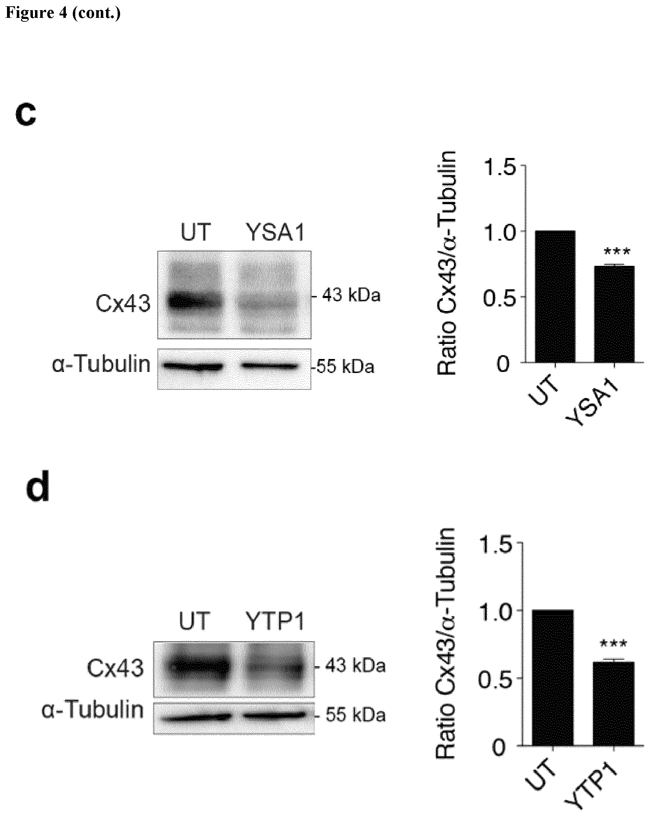

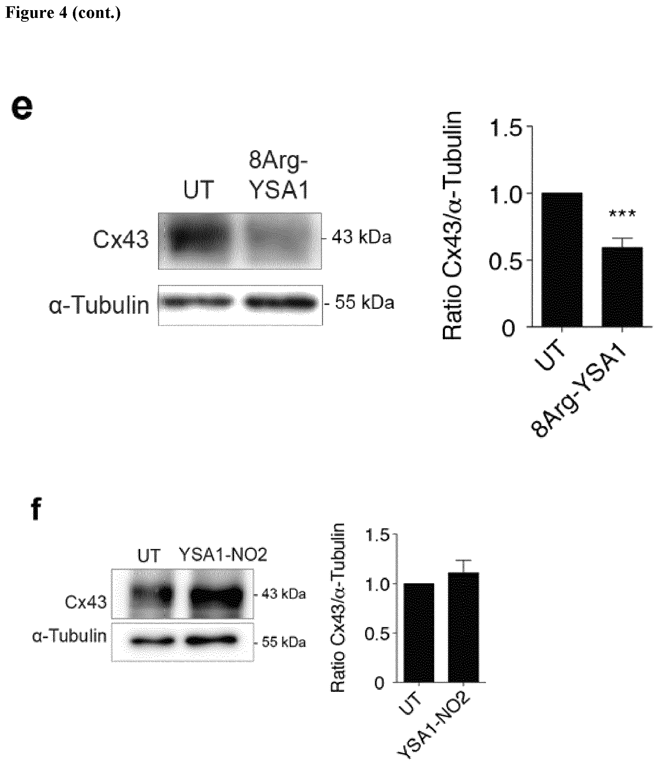

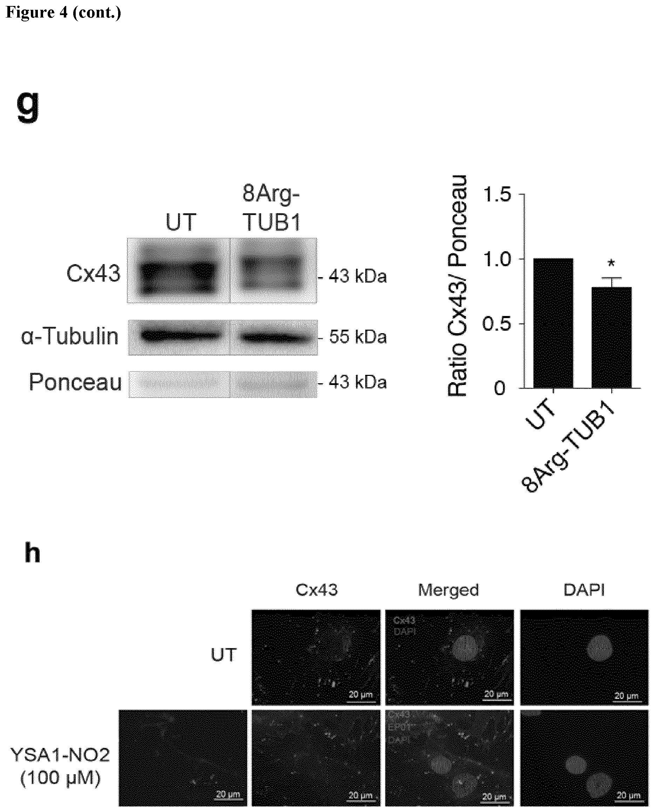

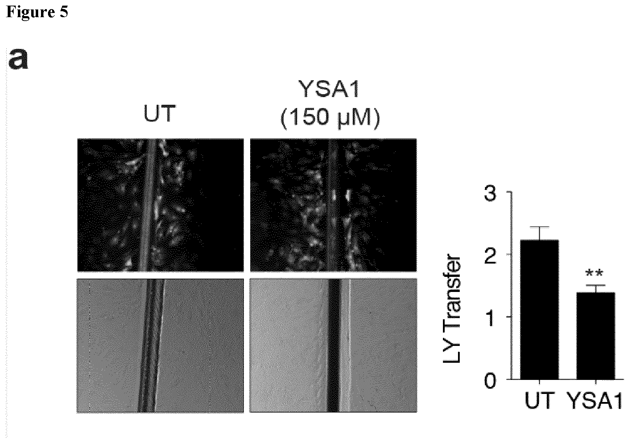

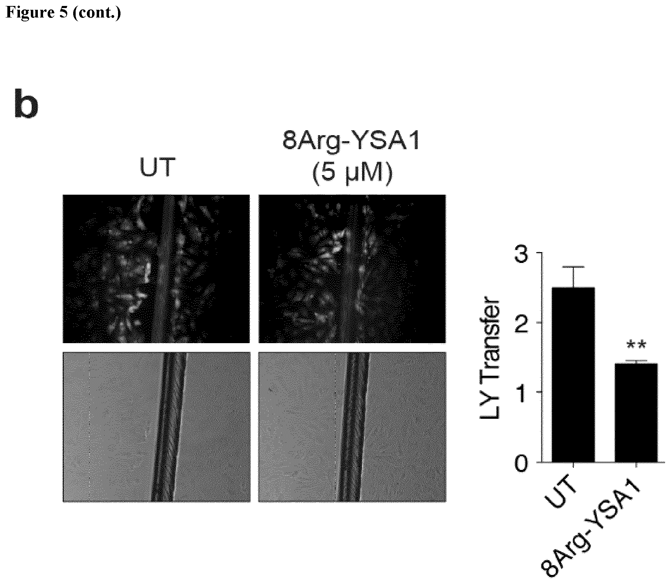

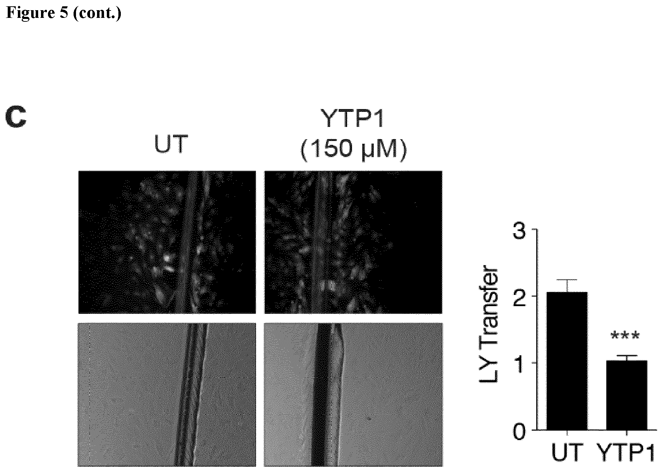

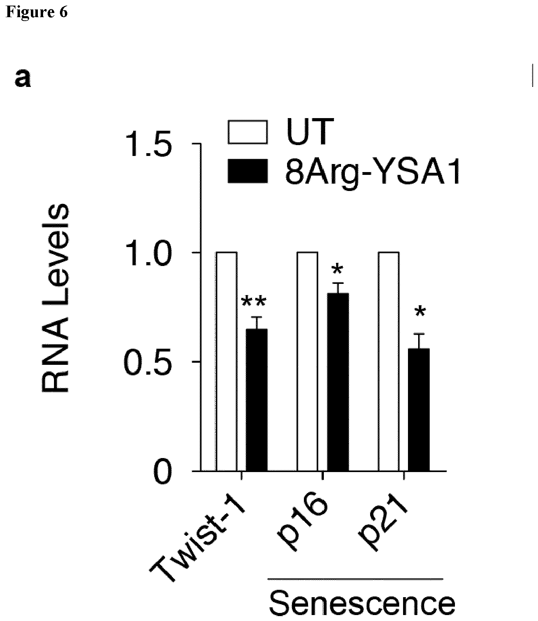

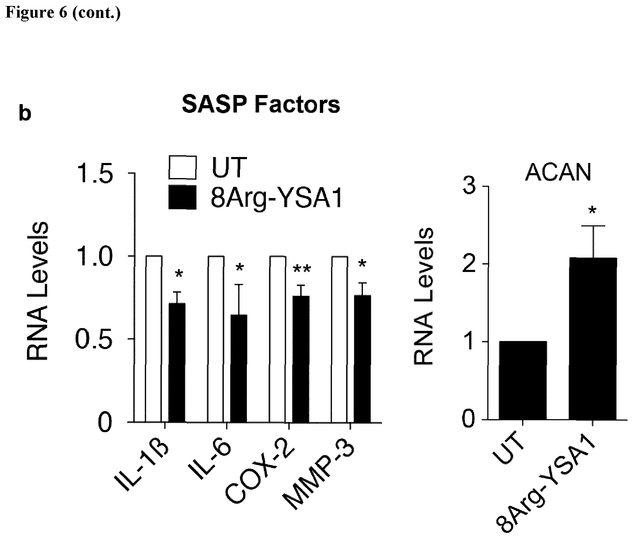

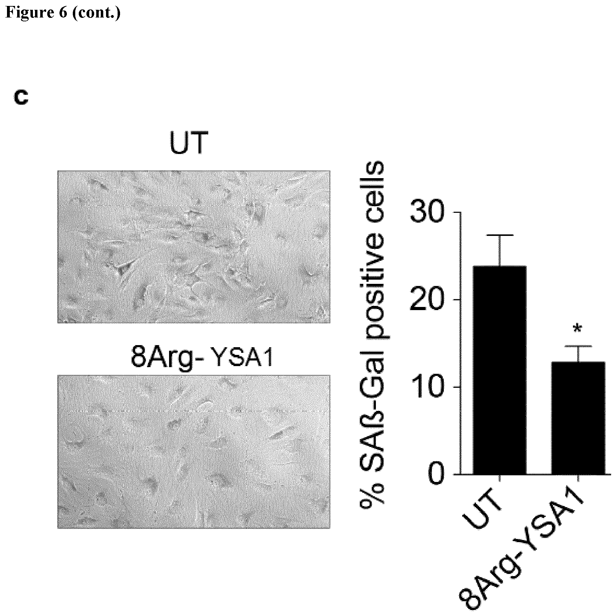

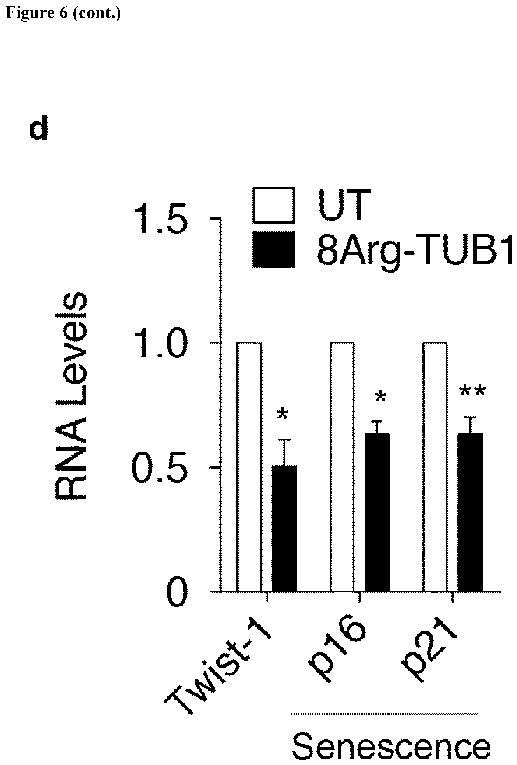

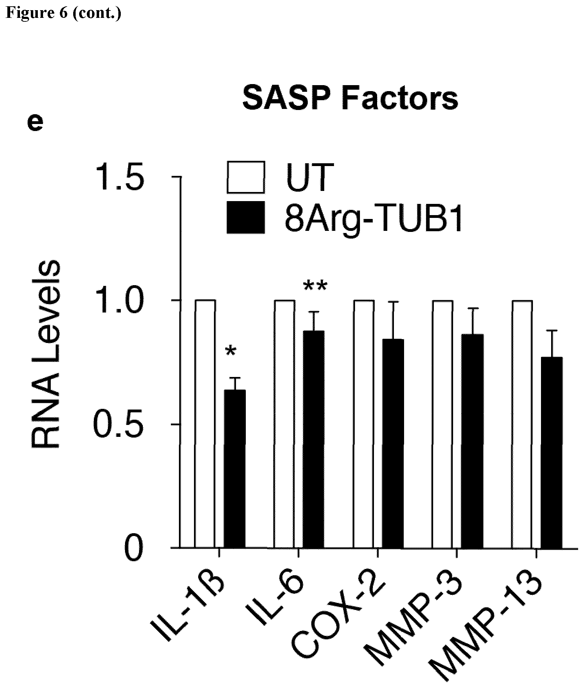

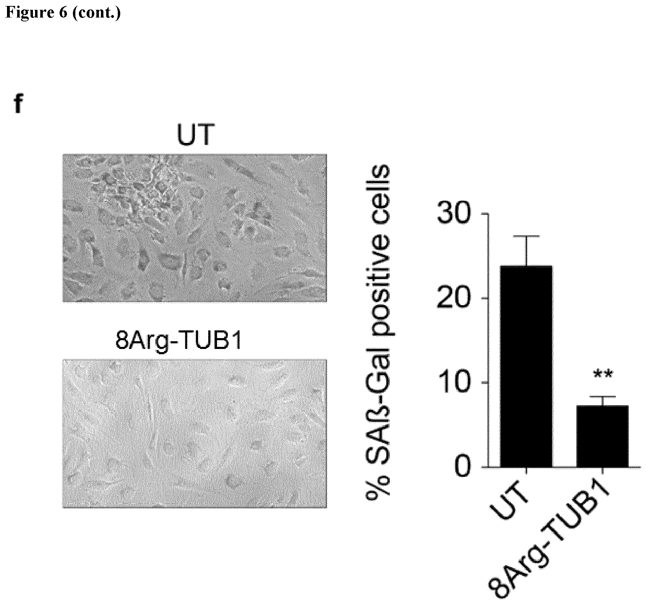

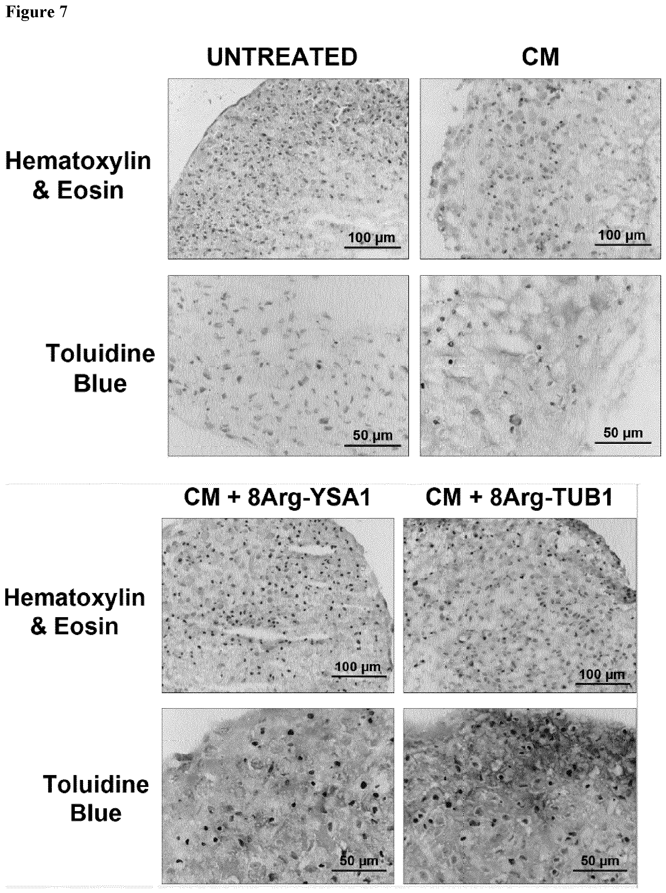

Such as can be seen in the results provided by the present invention:
- The peptides of the invention do not show toxicity (see **Example 2.1** and **Figure 2****).**
- The presence of the arginine tail (8-Arg) improves the internalization of the peptides in the cells (see **Example 2.2** and **Figure 3****).**
- The treatment with the peptides of the invention gives rise to a decrease in Cx43 levels in chondrocytes from patients with OA (Cx43 is significantly increased in the articular cartilage of patients suffering from OA and leads to senescence and dedifferentiation) (see **Example 2.3** and **Figure 4****).**
- The peptides of the invention decrease intercellular communication via gap junctions in OA chondrocytes (see **Example 2.4** and **Figure 5****).**
- The peptides of the invention improve the phenotype of the OA chondrocyte by increasing the expression of components and biomarkers of the extracellular matrix of the articular cartilage; decreasing the loss of phenotype involved in the progression of OA and reducing significantly the number of senescent cells (see **Example 2.5** and **Figure 6****).**
- The treatment with the peptides of the invention improves the composition and structure of the cartilage matrix secreted by chondrocytes in 3D cultures in micromasses (see **Example 2.6** and **Figure 7****).**

Thus, these peptides are useful for the treatment of OA or other aging-associated diseases that progress with accumulation of senescent cells. OA can used in the present invention as an illustrative example to prove the suitability of the peptides of the invention to i) reduce the accumulation of senescent cells, for example by inducing their death via apoptosis or inhibition of the SASP and/or ii) trigger the re-differentiation of cells once they have been de-differentiated. Nevertheless, this means that the peptides of the invention could be used for the prevention and treatment not only of OA, but of any other aging-associated disease associated with the persistence and accumulation of senescent cells for example (non-exhaustive list): obesity, renal disease, sarcopenia, fibrotic disease, atherosclerosis, cardiovascular disease, cancer, arthritis, cataracts, osteoporosis, type 2 diabetes or Alzheimer's disease. The incidence of all of these diseases increases exponentially with age [9].

Therefore, the first embodiment of the present invention refers to a senotherapeutic agent comprising at least one peptide selected from the list consisting of: SEQ ID NO: 1 or SEQ ID NO: 2.

In a preferred embodiment, the peptides comprise a N-terminal tail with 8 arginines.

The second embodiment of the invention refers to a pharmaceutical composition comprising the above mentioned senotherapeutic agent and, optionally, pharmaceutically acceptable excipients or carriers. In a preferred embodiment the peptides or the pharmaceutical composition are administered orally or by means of an intraarticular, subcutaneous, intra-venous or muscular injection.

The third embodiment of the present invention refers to a peptide selected from the list consisting of: SEQ ID NO: 1 or SEQ ID NO: 2 (or a pharmaceutical composition comprising said peptides), for use as a senotherapeutic agent, preferably in the prevention or treatment of osteoarthritis. In a preferred embodiment, the prevention or treatment of osteoarthritis is achieved by reducing the accumulation of chondrocyte senescent cells. In a preferred embodiment the prevention or treatment of osteoarthritis is achieved by triggering the re-differentiation of osteoarthritic chondrocytes once they have been de-differentiated.

For the purpose of the present invention the following definitions are provided:
- According to the present invention, "cellular senescence" refers to a natural biological state in which a cell permanently ceases cell division. Senescent cells accumulate with age and secrete more than 100 different biologically active molecules, including pro-inflammatory factors, proteases, pro-fibrotic factors and growth factors that disturb the tissue microenvironment. This secretome is referred to as the SASP. In addition to its effects on tissue function, the SASP contains factors that induce senescence, dedifferentiation and reprograming in neighbouring cells, setting off a cascade of events that culminates in the formation of the functionally aged and/or diseased tissue that appears to underlie the progression of a variety of age-related diseases. Thus, the elimination of senescent cells and modulation of SASP, address a root cause of the progression of diseases during aging.
- According to the present invention "senotherapeutic agents with regenerative capacity" are designed to selectively remove senescent cells and/or targeting the SASP. More specifically, the senotherapeutic agent is a molecule able to either i) reduce the accumulation of senescent cells for example by inducing their death via apoptosis or inhibition of the SASP component and ii) trigger the re-differentiation of cells once they have been de-differentiated. Both reduced levels of senescence and enhancement of re-differentiation restore the capacity of the tissues to restore regeneration. The purpose of the "senotherapeutic agents" is to delay, prevent, alleviate, or reverse age-related diseases which are caused by the persistence and accumulation of senescent cells as senescence via SASP also induces inflammation, reprogramming and dedifferentiation of neighbouring cells, being therefore one of the key factors involved in tissue degeneration and progression of aging-associated diseases [25, 26].

- "Pharmaceutically acceptable excipient or carrier" refers to an excipient that may optionally be included in the composition of the invention and that causes no significant adverse toxicological effects to the patient.
- By "therapeutically effective dose or amount" the present invention refers to the situation when the peptides or the composition are administered as described above and brings about a positive therapeutic response in a subject having an aging-associated disease. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein. Preferably, a therapeutically effective amount restores the function of the peripheral nerve.
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.

### Description of the figures

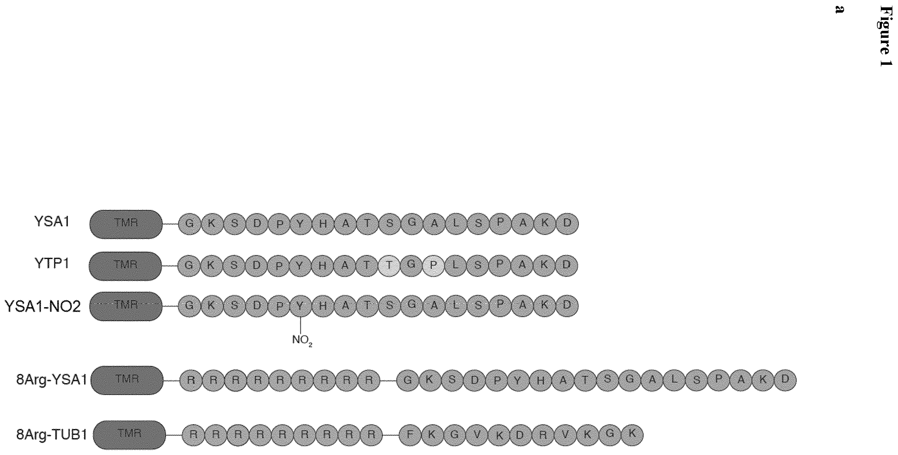

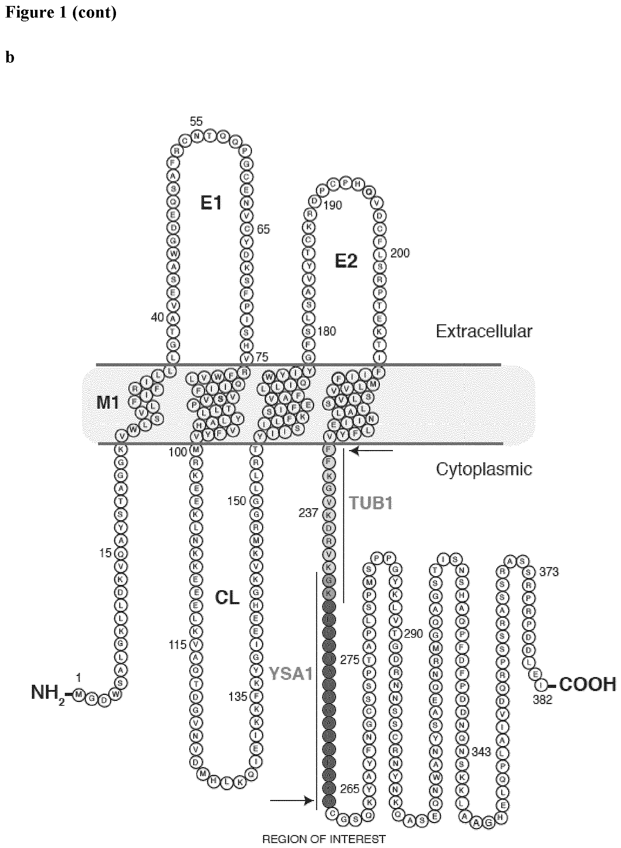

**Figure 1****. Representative diagram of the sequences corresponding to the peptides assayed in the present invention. (a)** Peptide YSA1 corresponds to residues 242-259 of the C-terminal domain (CTD) of Cx43, which include phosphorylation sites of Src (Y247) and MAPK (S255). The peptide YTP1 includes the same sequence, but with two mutations (S251T, A253P) that are found in the sequence of the pseudogene protein of Cx43 (GJA1P). A penetrating sequence of 8 arginines was added to the peptide YSA1 to facilitate its entry into the cell (8Arg-YSA1). Subsequently, YSA1 was mutated at the Y247 residue (Src) with a 3-nitrotyrosine residue to obtain the peptide YSA1-NO2. Peptide TUB1 corresponds to residues 232-243 of the CTD domain of Cx43, which includes microtubule binding sites (K234-K243). All peptides were labelled at their N-terminus with the TMR fluorophore to allow their detection by fluorescence. **(b)** Topological diagram of Cx43 showing the total region of interest from the residue F232-D265.
**Figure 2****. Cell viability assay to study toxicity.** A MTT viability assay was carried out in primary chondrocytes from articular cartilage of patients with OA treated with increasing concentrations of the peptide 8Arg-YSA1 (1-10-60-600 µM, with the octaarginine sequence and the TMR), for 16 hours. As indicated in the figure, the different concentrations studied did not affect cell viability with respect to the same untreated (NT) cells.
**Figure 3****. The presence of the octaarginine tail (8-Arg) improves the internalization of the YSA1 peptide sequence.** Chondrocytes at a confluence of 70-80% were treated for 16 hours with the peptides labelled with TMR for visualization at the cellular level at a concentration of 150 µM (YSA1, YTP1 and YSA1-NO2). The presence of the 8-Arg tail increases the penetrance of the peptide allowing the reduction of the effective concentration up to 30 times (5 µM), both in the case of the peptide 8Arg-YSA1 and 8Arg-TUB1. The images indicate that YSA1 and TUB1 are located mainly at the cytoplasmic level.
**Figure 4****. Decrease in Cx43 levels in chondrocytes from patients with OA treated with peptides YSA1, YTP1 and TUB1.** Patients with OA have significantly elevated levels of Cx43 in the articular cartilage activating cellular dedifferentiation via EMT by activation of Twist-1 and cellular senescence via p53/p16. The decrease in the levels and activity of Cx43 activates the re-differentiation of the chondrocyte and decreases the accumulation of senescent cells, restoring the adult chondrocyte phenotype and tissue regeneration capacity. **(a)** A decrease in positivity was detected for Cx43 in the OA chondrocytes by immunofluorescence studies for the detection of Cx43, especially in the membrane, or in the margin of the cells, when cells were treated with 200 µM of YSA1 or YTP1, and in comparison, with untreated (UT) cells **(a)** or with 5 µM of the peptide 8-Arg-YSA1 **(b).** The results obtained were confirmed by western-blot using α-tubulin as loading control. **(c)** Treatment with 150 µM YSA1 for 16 hours significantly reduced the levels of Cx43 in OA chondrocytes, compared to the same untreated (UT) cells. **(d)** Chondrocytes isolated from donors with osteoarthritis were treated for 16 hours with the YTP1 peptide (150 µM) and the Cx43 levels were analysed by western blot, using α-tubulin as loading control. As with the peptide YSA1, treatment with the peptide YTP1 reduced the levels of Cx43 in OA chondrocytes, compared to the same untreated (UT) cells. **(e)** Chondrocytes isolated from donors with OA were treated for 16 hours with the 8Arg-YSA1 peptide (5 µM) and the Cx43 levels were analysed by western blot, using α-tubulin as loading control. **(f)** The nitration of the Y247 residue of the YSA1 peptide prevented the decrease of Cx43 when the chondrocytes are treated with this modified peptide (YSA1-NO2) indicating the importance of this tyrosine for the activity of the YSA1 peptide and its potential application for the treatment of the OA. **(g)** Chondrocytes isolated from donors with OA were treated for 16 hours with the TUB1 peptide (5 µM) and the Cx43 levels were analysed by western blot, using the intensity of the Ponceau stain as loading control. As with the previous peptides, treatment with the TUB1 peptide reduced the Cx43 levels in OA chondrocytes, compared to the same untreated (UT) cells. **(h)** The immunofluorescence images confirm that the nitrated peptide YSA1-NO2 is able to cross the cell membrane but does not decrease the Cx43 levels (immunofluorescence).
**Figure 5****. Peptides YSA1 and YTP1 decrease intercellular communication through gap junctions (GJs) formed by Cx43 in OA chondrocytes. (a)** Scrape Loading / Dye Transfer (SL / DT) assay in which the transfer of Lucifer Yellow (LY) fluorophore between contact chondrocytes through GJs is studied. OA chondrocytes treated with peptide YSA1 (150 µM) for 16 hours showed a decrease in cell coupling compared to untreated (UT) cells. **(b)** The presence of 8-Arg decreased cell coupling mediated by this peptide at lower concentrations (5 µM, 16 h). **(c)** Treatment with YTP1 decreased cell coupling through GJs when the cells were treated with 150 µM YTP1 for 16 hours.
**Figure 6****. The peptides of this invention improve OA chondrocyte phenotype by decreasing senescence involved in OA progression and increasing the expression of cartilage extracellular matrix proteins.** OA chondrocytes show increased Cx43 levels associated to EMT and senescence via Twist-1 and p53/p21/p16, respectively. In addition, senescent cell accumulation is involved in OA progression. **(a)** The treatment with the 8Arg-YSA1 peptide (5 µM) for 16 hours led to reduced expression of Twist-1, a transcription factor that activates cell dedifferentiation via EMT, and the senescence-activation factors p16 and p21. RNA levels of Twist-1, p16 and p21 were normalized to HPRT1 levels and compared to untreated OACs (osteoarthritis chondrocytes) (UT). **(b)** SnCs share common features that are extensively used to detect and/or target this type of cells. Within the commonly shared biomarkers, SnCs display enhanced acidic lysosomal β-galactosidase activity (SA-β-Gal), increased expression of the cyclin-dependent kinase inhibitors p21^{CIP} and p16^{INK4A}, and enhanced secretion of pro-inflammatory and tissue-remodelling factors within the SASP. Gene expression analysis of OACs treated with 8Arg-YSA1 (5 µM) for 16 hours confirmed the downregulation of SASP factors including proinflammatory cytokines (IL-1β, IL-6, COX-2) and matrix-degrading enzymes such as metalloprotease-3 (MMP-3) and increased expression of cartilage ECM markers, such as aggrecan (ACAN). RNA levels were normalized to HPRT1 levels and compared to untreated OA chondrocytes (UT). **(c)** Treatment of OACs for 7 days with the peptide 8Arg-YSA1 (5 µM) reduced significantly the number of senescent cells detected by β-galactosidase staining. **(d)** The treatment with the 8Arg-TUB1 peptide (5 µM) for 16 hours also lead to reduced expression Twist-1 and the senescence-activation factors p16 and p21 in OACs. **(e)** In addition, OACs treated with 8Arg-TUB1 (5 µM) for 16 hours lead to diminished expression of SASP factors IL-1β, IL-6, COX-2 and MMP-3. **(f)** Treatment of OACs for 7 days with the peptide 8Arg-TUB1 (5 µM) reduced significantly the number of senescent cells detected by β-galactosidase staining.
**Figure 7****. YSA1 and TUB1 improve the composition and structure of the extracellular matrix of chondrocytes cultured as micromasses.** Chondrocytes isolated from OA donors were cultured as 3D micromasses for 14 days in normal growth medium or in chondrogenic medium (CM) alone or supplemented with 5 µM of the peptides 8Arg-YSA1 or 8Arg-TUB1. Hematoxylin-Eosin stain showed that micromasses treated with these peptides lead to a better ECM structure. In addition, blue toluidine stain showed an increased deposition of proteoglycans in the ECM of micromasses treated with the peptides 8Arg-YSA1 or 8Arg-TUB1, as detected by the increased violet dye.

### Detailed description of the invention

### Example 1. Material and Methods.

### Example 1.1. Primary culture.

The primary cultures of chondrocytes were obtained from articular cartilage of the hip and knee from patients with OA and healthy individuals obtained in the *Complexo Hospitalario Universitario de Santiago de Compostela* (CHUS-XXIS) under informed consent and approved by the Institutional Ethics Committee within the private collection of biological samples of the group (C.0003333, 2012/094 and 2015/029). With the help of a scalpel, the cartilage was separated from the bone and divided into small fragments of 1 mm. The fragments were then digested enzymatically with trypsin for 10 min at 37°C under stirring, followed by digestion with collagenase IV at 37°C under stirring for 16 hours. Subsequently, the sample was passed through a 100 µM filter and the cells were seeded in Dulbecco's modified Eagle medium medium (DMEM, Lonza) supplemented with 10% foetal bovine serum (SFB, Gibco, Thermo Scientific) and 1% Penicillin (100 U / ml) / Streptomycin (100 µg / ml) (Gibco, Thermo Scientific) and kept in culture in an incubator at 37°C, 5% CO₂ and humidity saturation.

### Example 1.2. Synthesis of peptides.

The tested peptides were synthesized in Centro Singular de Investigación en Química Biolóxica e Materiais Moleculares (CIQUS) and Centro de Investigacións Científicas Avanzadas (CICA). The YSA1 peptide sequence corresponds to part of the C-terminal domain of Cx43 and contains a phosphorylation site of the Src kinase (Y247) and another phosphorylation site (S255) of the mitogen-activated protein kinase (Mitogen-Activated Potein Kinase, MAPK). A new peptide of similar sequence to YSA1 was synthesized but mutated in two positions and designated as YTP1 **(****Figure 1****,** YSA1-mutated). Both peptides contain a sequence of 8 arginines at the N-terminus (8Arg) and a fluorescent molecule to study their subcellular location (tetramethylrhodamine, TMR). Finally, YSA1-NO2 contains a 3-nitrotyrosine residue in the same position than the tyrosine residue phosphorylated by Src (Y247), to study the effect of tyrosine nitration on the YSA1 peptide (see **Figure 1****).** Peptides were synthesized following standard Fmoc solid phase peptide synthesis (SPPS) protocols. After completing the synthesis of the peptide sequences, their N-terminus were deprotected by treatment with 20% 4-methylpiperidine in N,N-dimethylformamide (DMF). Then, the fluorophore was attached to the *N*-terminus of the peptides by treatment of the deprotected resins with 6-Carboxytetramethylrhodamine succinimidyl ester in presence of *N,N-*diisopropylethylamine (DIEA) and DMF. Once the fluorophore was coupled to the peptides, those were cleaved from the resin and the corresponding residues were purified by reversed-phase HPLC, and the fractions identified as the desired products were lyophilized.

In a preferred embodiment the peptides can comprise a fluorophore like tetramethylrhodamine (TAMRA) linked to aminohexanoic acid (Ahx), or a fluorophore like tetramethylrhodamine (TAMRA) linked to a tail of 8 arginines and also to the aminohexanoic acid (Ahx).

### Example 1.3. MTT cell viability assay.

An initial number of cells (5,000-10,000) were seeded in a 96-well culture plate and treated with different concentrations of the peptides. After the treatment, the medium was removed from each well and 10 µL of the MTT reagent was added to 100 µL of fresh medium and incubated at 37°C for 4 hours. To solubilize the formazan crystals of the MTT reagent, it was incubated with DMSO at room temperature and under stirring for 30 minutes. Absorbance was measured at 570 nm in a plate reader (Nanoquant Infinite M200, TECAN).

### Example 1.4. Visualization of peptide internalization.

Primary cultures of chondrocytes at a confluence of 70-80% were treated with the corresponding peptide. After 16 hours, the subcellular localization of the TMR-labeled peptides was visualized *in vivo* in a fluorescence inverted microscope (Inverted Research Microscope Eclipse Ti, Nikon).

### Example 1.5. Immunofluorescence.

Primary cultures of chondrocytes at a confluence of 70-80% were treated with mimetic peptides of Cx43, and fixed with 2-4% paraformaldehyde (PFA, Sigma-Aldrich) in phosphate buffered saline (PBS, MP Biomedicals) for 10 minutes at room temperature. The PFA was removed and cells were incubated in 0.1 M glycine (Sigma-Aldrich) for 10 minutes to remove autofluorescence from the PFA, and the cell membranes were permeabilized with 0.2% Triton X-100 (Sigma-Aldrich) in PBS for 10 minutes. The non-specific binding sites were blocked with 1% bovine serum albumin (BSA, Sigma-Aldrich) in PBS with Tween-20 (PBS-T, Sigma-Aldrich), for 30 minutes. Subsequently, the cells were incubated with the primary anti-Cx43 antibody (C6219, Sigma-Aldrich) for 1 hour, at room temperature and washed with PBS for 10 minutes to remove excess antibody. It was incubated with the secondary antibody labelled with FITC (anti-rabbit F2765, Thermo Fisher) for 1 hour at room temperature, in the dark. Excess antibody was removed, and the nuclei were stained with 4 ', 6-diamino-2-phenylindole (DAPI, Sigma-Aldrich) for 4 minutes. Finally, preparations were mounted with a drop of glycergel aqueous mounting medium (Dako) and visualized in an Olympus BX61 fluorescence microscope using a DP71 digital camera (Olympus).

### Example 1.6. Western-blot.

The cell pellet isolated from patients with OA and treated with the peptides or untreated were mechanically and chemically lysed using a needle of 29 gauges (BD Medical) in the presence of a cold lysis buffer (50 mM Tris HCl pH 7.5; 50 mM EDTA pH 8, 0.5% NP-40, 1% Triton X-100, 150 mM NaCl). Once the cell extracts were obtained, they were diluted in loading buffer (10% SDS, 200 mM Tris HCl pH 6.8, 50% glycerol, 0.1% Bromophenol Blue, 10% β-Mercaptoethanol). Equal amounts of the cell extracts were separated on a 10% Acrylamide / Bisacrylamide (Acr / Bis) gel with SDS. The proteins were subsequently transferred to a polyvinylidene fluoride membrane (PVDF, Merck) in a Trans-Blot^{®} SD Semi-Dry Transfer Cell (Bio-Rad). It was verified that the transfer was correct by staining the membrane with ATX Ponceau S (Sigma-Aldrich). To block possible nonspecific binding with the membrane, 1-hour incubation was performed at room temperature with a 5% milk solution in a TTBS buffer (20 mM Tris, 150 mM NaCl, 0.05% Tween-20). The membrane was incubated with the primary antibody at 4°C under stirring for 16 hours. The excess antibody was removed with successive washes with TTBS and then incubated with the secondary antibody at room temperature under stirring for 1 hour. Excess antibody was removed, and the signal was developed with the commercial kit of Pierce^{™} ECL Western Blotting Substrate (Thermo Fisher Scientific) in an Amersham Imager 600 developing chamber (GE Healthcare). The primary antibodies used were anti-Cx43 (C6219, Sigma-Aldrich) and anti-α-tubulin (T9026 Sigma-Aldrich). The secondary antibodies used were a goat anti-rabbit (A6154 Sigma-Aldrich) and a sheep anti-mouse (NA-931 Sigma-Aldrich), respectively.

### Example 1.7. Scrape Loading / Dye Transfer (SL / DT) assay.

Cells were seeded in culture plates to an approximate 90% confluence. After treating the cells with the peptides, the medium was removed and washed twice with PBS. The fluorescent compound Lucifer Yellow (LY, Cell Projects Ltd ^{©} Kent) 0.4% in PBS was added and two cuts were made, one with a scalpel and the other with a needle on the cell monolayer. The cells were incubated for 5-7 minutes at 37°C to allow the transfer of the LY between undamaged cells in contact, after which the LY was removed, and cells were washed with PBS. The cells were fixed with 4% formaldehyde for 10 minutes. The visualization was performed in an inverted fluorescence microscope (Inverted Research Microscope Eclipse Ti, Nikon).

### Example 1.8. Gene expression analysis (qPCR).

After treatments with the peptides, the cells were trypsinized and the pellets were lysed in TRI Reagent (MRC), following the manufacturer's instructions until obtaining an RNA pellet. Subsequently, a treatment with 1 U / µl of DNAases was performed to eliminate contaminating DNA. The quality and quantity of RNA isolated was quantified in a Nanodrop ND-1000 spectrophotometer (Thermo Fisher Scientific). For retro-transcription of the RNA and obtaining the complementary DNA, 1 µg of RNA and the Superscript^{®} VILOTM Master Mix commercial kit (Invitrogen) were used in a Veriti thermocycler (Applied Biosystems) with the following program: a 10-minute denaturation 65°C, followed by 10 minutes at 25°C, an incubation of 60 minutes at 42°C and 5 minutes at 85°C. The complementary DNA was diluted in water free of proteases, DNAases and RNAases (Sigma-Aldrich). Finally, quantitative real-time PCR (qPCR) was carried out using the PowerUp^{™} SYBR^{®} Green Master Mix (Thermo Fisher Scientific) in the LightCycler^{®} 480 Instrument (Roche, Applied Science) with the following program: 10 minutes incubation at 95°C, followed by amplification of 30-40 cycles from 10 to 95°C, 30 to 60°C and 12 to 72°C. *HPRT-1* was used as a housekeeping gene, and the levels of gene expression were analysed with respect to the control cells without treatment. The primers used were the following:

**Table 1**

| Gene | Forward | Reverse |
|---|---|---|
| *HPRT-1* | SEQ ID NO: 4 | SEQ ID NO: 5 |
| *CDKN2A* (p 16) | SEQ ID NO: 6 | SEQ ID NO: 7 |
| *CDKN1A* (p21) | SEQ ID NO: 8 | SEQ ID NO: 9 |
| *MMP-3* | SEQ ID NO: 10 | SEQ ID NO: 11 |
| *TWIST-1* | SEQ ID NO: 12 | SEQ ID NO: 13 |
| *ACAN* | SEQ ID NO: 14 | SEQ ID NO: 15 |
| *IL-1β* | SEQ ID NO: 16 | SEQ ID NO: 17 |
| *IL-6* | SEQ ID NO: 18 | SEQ ID NO: 19 |
| *PTGS2* (COX-2) | SEQ ID NO: 20 | SEQ ID NO: 21 |

### Example 1.9. Chondrogenic differentiation.

Primary chondrocytes isolated from donors with osteoarthritis were cultured as three-dimensional micromasses. For this, 500,000 cells were seeded in polystyrene tubes and centrifuged at 1500 rpm for 10 minutes, forming a cell pellet. Then, the medium was replaced by the corresponding treatment: untreated control (DMEM 10% FBS), or chondrogenic medium alone or supplemented with 5 µM 8Arg-YSA1 or 8Arg-TUB1. The medium was changed every 2-3 days, and after 14 days, the micromasses were frozen in Tissue-Tek^{®} O.C.T. (Sakura Finetek).

### Example 1.10. Detection of senescence-associated β-galactosidase activity (SA-β-Gal).

For the detection of the senescence-associated β-galactosidase activity we have used the Senescence Cells Histochemical Staining kit (Sigma-Aldrich). Cells were rinsed with warm PBS and fixed for 7 minutes at room temperature with a buffer composed by 2% paraformaldehyde and 0.2% glutaraldehyde. After three washes with PBS, cells were incubated 16 h at 37°C without CO₂ in a staining solution containing X-gal, which is cleaved by the β-galactosidase enzyme producing an intense blue insoluble product.

### Example 2. Results.

### Example 2.1. Cell viability assay to study toxicity.

Such as it is explained in **Figure 2****,** a MTT viability assay was carried out on primary chondrocytes from articular cartilage of patients with OA treated with increasing concentrations of the peptide 8Arg-YSA1 (1-10-60-600 µM, with the arginine sequence and the TMR), for 16 hours.

The different concentrations studied did not affect cell viability with respect to the same untreated (UT) cells.

### Example 2.2. The presence of the octaarginine tail (8-Arg) improves the internalization of the YSA1 peptide sequence.

Chondrocytes at a confluence of 70-80% were treated for 16 hours with the peptides labelled with TMR for visualization at the cellular level at a concentration of 150 µM (YSA1, YTP1 and YSA1-NO2) (see **Figure 3****).** The presence of the 8-Arg tail increases the penetrance thus giving the possibility of reducing the concentrations of the peptide up to 30 times (5 µM) both in the case of the peptide 8Arg-YSA1 and 8Arg-TUB1. The images depicted in **Figure 3** indicate that YSA1 and TUB1 are located mainly at the cytoplasmic level.

### Example 2.3. Decrease in Cx43 levels in chondrocytes from patients with OA treated with peptides YSA1, YTP1 and TUB1.

Patients with OA have significantly elevated levels of Cx43 in the articular cartilage activating cellular dedifferentiation via EMT by activation of Twist-1 and cellular senescence via p53/p16.

Such as it is shown in **Figure 4****,** peptides YSA1, YTP1 and TUB1 were able to decrease the levels and activity of Cx43. By targeting Cx43, these peptides activate the re-differentiation of the chondrocyte and decrease the accumulation of senescent cells, restoring the adult chondrocyte phenotype and the capacity for tissue regeneration.

Such as it is shown in **Figure 4****,** a decrease in positivity was detected for Cx43 in the OA chondrocytes, especially in the membrane, or in the margin of the cells (channel activity, GJs), when they were treated with 200 µM of YSA1 or YTP1, against the untreated (UT) cells **(****Figure 4a****)** or with 5 µM of the peptide 8Arg-YSA1 **(****Figure 4b****).** The results obtained were confirmed by western-blot using α-tubulin as loading control. Moreover, treatment with 150 µM YSA1 for 16 hours significantly reduced the levels of Cx43 in OA chondrocytes, compared to the same untreated (UT) cells **(****Figure 4c****).** On the other hand, chondrocytes isolated from donors with osteoarthritis were treated for 16 hours with the YTP1 peptide (150 µM) and the Cx43 levels were analysed by western blot, using α-tubulin as loading control **(****Figure 4d****).** As with the peptide YSA1, treatment with the peptide YTP1 reduced the levels of Cx43 in OA chondrocytes, compared to the same untreated (UT) cells. Moreover, chondrocytes isolated from donors with osteoarthritis were treated for 16 hours with the 8Arg-YSA1 peptide (5 µM) and the Cx43 levels were analysed by western blot, using α-tubulin as loading control **(****Figure 4e****).** The nitration of the Y247 residue of the YSA1 peptide prevented the decrease of Cx43 when the chondrocytes are treated with this modified peptide (YSA1-NO2) indicating the importance of this tyrosine for the activity of the YSA1 peptide and its potential application for the treatment of the OA **(****Figure 4f****).** Chondrocytes isolated from donors with osteoarthritis were treated for 16 hours with the 8Arg-TUB1 peptide (5 µM) and the Cx43 levels were analysed by western blot, using the intensity of the Ponceau stain as loading control. As with the previous peptides, treatment with the 8Arg-TUB1 peptide reduced the Cx43 levels in OA chondrocytes, compared to the same untreated (UT) cells **(****Figure 4g****).** The immunofluorescence images **(****Figure 4h****)** confirm that the nitrated peptide YSA1-NO2 is able to cross the cell membrane but does not decrease the Cx43 levels (immunofluorescence).

### Example 2.4. The peptides YSA1 and YTP1 impair intercellular communication through communicating junctions (GJs) in OA chondrocytes.

A Scrape Loading / Dye Transfer (SL/DT) test was performed in which the transfer of Lucifer Yellow fluorophore (LY) between contact chondrocytes through GJs was studied. OA chondrocytes treated with peptide YSA1 (150 µM) for 16 hours showed a decrease in cell coupling compared to untreated (UT) cells **(****Figure 5a****).** The presence of 8-Arg made it possible to decrease cell coupling more efficiently at lower concentrations (5 µM 8Arg-YSA1, 16 hours) **(****Figure 5b****).** Treatment with YTP1 decreased cell coupling through GJs when the cells were treated with 150 µM for 16 hours **(****Figure 5c****).**

### Example 2.5. Peptides under study improve the phenotype of the OA chondrocyte by decreasing the senescence involved in the progression of OA and increasing the expression of components of the extracellular matrix of the articular cartilage.

OA chondrocytes show increased Cx43 levels that lead to EMT and senescence via Twist-1 and p53/p21/p16, respectively. In addition, senescent cell accumulation is involved in OA progression. The treatment with the 8Arg-YSA1 peptide (5 µM) for 16 hours lead to reduced expression Twist-1, a transcription factor that activate cell dedifferentiation via EMT, and the senescence-activation factors p16 and p21 **(****Figure 6a****).** RNA levels of Twist-1, p16 and p21 were normalized to HPRT1 levels and compared to untreated OACs (UT). Gene expression analysis of OACs treated with 8Arg-YSA1 (5 µM) for 16 hours confirmed the downregulation of SASP factors including proinflammatory cytokines (IL-1β, IL-6, COX-2) and matrix-degrading enzymes such as metalloprotease-3 (MMP-3) and increased expression of cartilage ECM markers, such as aggrecan (ACAN). RNA levels were normalized to HPRT1 levels and compared to untreated OACs (UT) **(****Figure 6b****).** Treatment of OACs for 7 days with the peptide 8Arg-YSA1 (5 µM) reduced significantly the number of senescent cells detected by β-galactosidase staining **(****Figure 6c****).** The treatment with the 8Arg-TUB1 peptide (5 µM) for 16 hours also lead to reduced expression Twist-1 and the senescence-activation factors p16 and p21 in OACs **(****Figure 6d****).** In addition, OACs treated with 8Arg-TUB1 (5 µM) for 16 hours lead to diminished expression of SASP factors IL-1β, IL-6, COX-2, MMP-3 and MMP-13 **(****Figure 6e****).** Treatment of OACs for 7 days with the peptide 8Arg-TUB1 (5 µM) reduced significantly the number of senescent cells detected by β-galactosidase staining **(****Figure 6f****).**

### Example 2.6. The treatment with the peptides 8Arg-YSA1 and 8Arg-TUB1 improves the composition and structure of the extracellular matrix of chondrocytes cultured as micromasses.

Chondrocytes isolated from OA donors were cultured as 3D micromasses for 14 days in normal growth medium or in chondrogenic medium (CM) alone or supplemented with 5 µM of peptides 8Arg-YSA1 or 8Arg-TUB1. Hematoxylin-Eosin stain showed that micromasses treated with these peptides lead to a better ECM structure. In addition, blue toluidine stain showed an increased deposition of proteoglycans in the ECM of micromasses treated with the peptides 8Arg-YSA1 or 8Arg-TUB1, as detected by the increased violet dye (see **Figure 7****).**

### REFERENCES

1. Loeser RF. The Role of Aging in the Development of Osteoarthritis. Transactions of the American Clinical and Climatological Association. 2017; 128:44-54.
2. Collins JA, Diekman BO and Loeser RF. Targeting aging for disease modification in osteoarthritis. Curr Opin Rheumatol. 2018; 30(1):101-107.
3. Martel-Pelletier J, Barr AJ, Cicuttini FM, Conaghan PG, Cooper C, Goldring MB, Goldring SR, Jones G, Teichtahl AJ and Pelletier JP. Osteoarthritis. Nature reviews Disease primers. 2016; 2:16072.
4. Cross M, Smith E, Hoy D, Nolte S, Ackerman I, Fransen M, Bridgett L, Williams S, Guillemin F, Hill CL, Laslett LL, Jones G, Cicuttini F, et al. The global burden of hip and knee osteoarthritis: estimates from the global burden of disease 2010 study. Ann Rheum Dis. 2014; 73(7):1323-1330.
5. Storheim K and Zwart JA. Musculoskeletal disorders and the Global Burden of Disease study. Ann Rheum Dis. 2014; 73(6):949-950.
6. Varela-Eirin M, Varela-Vazquez A, Guitian-Caamano A, Paino CL, Mato V, Largo R, Aasen T, Tabernero A, Fonseca E, Kandouz M, Caeiro JR, Blanco A and Mayan MD. Targeting of chondrocyte plasticity via connexin43 modulation attenuates cellular senescence and fosters a pro-regenerative environment in osteoarthritis. Cell death & disease. 2018; 9(12):1166.
7. Jeon OH, Kim C, Laberge RM, Demaria M, Rathod S, Vasserot AP, Chung JW, Kim DH, Poon Y, David N, Baker DJ, van Deursen JM, Campisi J, et al. Local clearance of senescent cells attenuates the development of post-traumatic osteoarthritis and creates a pro-regenerative environment. Nat Med. 2017; 23(6):775-781.
8. Jeon OH, Wilson DR, Clement CC, Rathod S, Cherry C, Powell B, Lee Z, Khalil AM, Green JJ, Campisi J, Santambrogio L, Witwer KW and Elisseeff JH. Senescence cell-associated extracellular vesicles serve as osteoarthritis disease and therapeutic markers. JCI insight. 2019; 4(7).
9. Munoz-Espin D and Serrano M. Cellular senescence: from physiology to pathology. Nat Rev Mol Cell Biol. 2014; 15(7):482-496.
10. Price JS, Waters JG, Darrah C, Pennington C, Edwards DR, Donell ST and Clark IM. The role of chondrocyte senescence in osteoarthritis. Aging cell. 2002; 1(1):57-65.
11. Philipot D, Guerit D, Platano D, Chuchana P, Olivotto E, Espinoza F, Dorandeu A, Pers YM, Piette J, Borzi RM, Jorgensen C, Noel D and Brondello JM. p16INK4a and its regulator miR-24 link senescence and chondrocyte terminal differentiation-associated matrix remodeling in osteoarthritis. Arthritis Res Ther. 2014; 16(1):R58.
12. Gao SG, Zeng C, Li LJ, Luo W, Zhang FJ, Tian J, Cheng C, Tu M, Xiong YL, Jiang W, Xu M and Lei GH. Correlation between senescence-associated beta-galactosidase expression in articular cartilage and disease severity of patients with knee osteoarthritis. International journal of rheumatic diseases. 2016; 19(3):226-232.
13. Tchkonia T, Zhu Y, van Deursen J, Campisi J and Kirkland JL. Cellular senescence and the senescent secretory phenotype: therapeutic opportunities. J Clin Invest. 2013; 123(3):966-972.
14. Varela-Eirin M, Loureiro J, Fonseca E, Corrochano S, Caeiro JR, Collado M and Mayan MD. Cartilage regeneration and ageing: Targeting cellular plasticity in osteoarthritis. Ageing research reviews. 2017; 42:56-71.
15. Gangoso E, Talaveron R, Jaraiz-Rodriguez M, Dominguez-Prieto M, Ezan P, Koulakoff A, Medina JM, Giaume C and Tabernero A. A c-Src Inhibitor Peptide Based on Connexin43 Exerts Neuroprotective Effects through the Inhibition of Glial Hemichannel Activity. Frontiers in molecular neuroscience. 2017; 10:418.
16. Varela-Vazquez A, Guitian-Caamano A, Carpintero-Fernandez P, Fonseca E, Sayedyahossein S, Aasen T, Penuela S and Mayan MD. Emerging functions and clinical prospects of connexins and pannexins in melanoma. Biochimica et biophysica acta Reviews on cancer. 2020; 1874(1):188380.
17. Leithe E, Mesnil M and Aasen T. The connexin 43 C-terminus: A tail of many tales. Biochim Biophys Acta. 2018; 1860(1):48-64.
18. Aasen T, Mesnil M, Naus CC, Lampe PD and Laird DW. Gap junctions and cancer: communicating for 50 years. Nat Rev Cancer. 2016; 16(12):775-788.
19. Rhett JM, Calder BW, Fann SA, Bainbridge H, Gourdie RG and Yost MJ. Mechanism of action of the anti-inflammatory connexin43 mimetic peptide JM2. Am J Physiol Cell Physiol. 2017; 313(3):C314-C326.
20. Ghatnekar GS, O'Quinn MP, Jourdan LJ, Gurjarpadhye AA, Draughn RL and Gourdie RG. Connexin43 carboxyl-terminal peptides reduce scar progenitor and promote regenerative healing following skin wounding. Regenerative medicine. 2009; 4(2):205-223.
21. Jaraiz-Rodriguez M, Talaveron R, Garcia-Vicente L, Pelaz SG, Dominguez-Prieto M, Alvarez-Vazquez A, Flores-Hernandez R, Sin WC, Bechberger J, Medina JM, Naus CC and Tabernero A. Connexin43 peptide, TAT-Cx43266-283, selectively targets glioma cells, impairs malignant growth, and enhances survival in mouse models in vivo. Neuro-oncology. 2020; 22(4):493-504.
22. Lee AC, Harris JL, Khanna KK and Hong JH. A Comprehensive Review on Current Advances in Peptide Drug Development and Design. Int J Mol Sci. 2019; 20(10).
23. Lau JL and Dunn MK. Therapeutic peptides: Historical perspectives, current development trends, and future directions. Bioorganic & medicinal chemistry. 2018; 26(10):2700-2707.
24. Bruzzoni-Giovanelli H, Alezra V, Wolff N, Dong CZ, Tuffery P and Rebollo A. Interfering peptides targeting protein-protein interactions: the next generation of drugs? Drug discovery today. 2018; 23(2):272-285.
25. van Deursen JM. Senolytic therapies for healthy longevity. Science. 2019; 364(6441):636-637.
26. Kirkland JL and Tchkonia T. Clinical strategies and animal models for developing senolytic agents. Experimental gerontology. 2015; 68:19-25.

## Claims

1. Peptide consisting of SEQ ID NO: 1 or SEQ ID NO: 2.

2. Pharmaceutical composition comprising the peptide of claim 1 and, optionally, pharmaceutically acceptable excipients or carriers.

3. Peptide consisting of SEQ ID NO: 1 or SEQ ID NO: 2 for use as a therapeutic agent.

4. Peptide consisting of SEQ ID NO: 1 or SEQ ID NO: 2 for use, according to claim 3, in the prevention or treatment of osteoarthritis.

5. Peptide consisting of SEQ ID NO: 1 or SEQ ID NO: 2 and further comprising a *N-*terminal tail of 8 arginines, for use according to claims 3 or 4.

6. Peptide for use, according to any of the claims 3 to 5, wherein the peptide is administered orally or by means of an intraarticular, subcutaneous, intra-venous or muscular injection.

## Patentansprüche

1. Peptid bestehend aus SEQ ID NO: 1 oder SEQ ID NO: 2.

2. Pharmazeutische Zusammensetzung umfassend das Peptid nach Anspruch 1 und, wahlweise, pharmazeutisch akzeptable Hilfsstoffe oder Trägerstoffe.

3. Peptid bestehend aus SEQ ID NO: 1 oder SEQ ID NO: 2 zur Verwendung als Therapeutikum.

4. Peptid bestehend aus SEQ ID NO: 1 oder SEQ ID NO: 2 zur Verwendung, nach Anspruch 3, bei der Verbeugung oder Behandlung von Osteoarthritis.

5. Peptid bestehend aus SEQ ID NO: 1 oder SEQ ID NO: 2 und zusätzlich umfassend einen N-Terminus aus 8 Argininen, zur Verwendung nach den Ansprüchen 3 oder 4.

6. Peptid zur Verwendung, nach einem der Ansprüche 3 bis 5, wobei das Peptid oral oder mittels einer intraartikulären, subkutanen, intravenösen oder muskulären Spritze verabreicht wird.

## Revendications

1. Peptide consistant en SEQ ID NO : 1 ou SEQ ID NO : 2.

2. Composition pharmaceutique comprenant le peptide selon la revendication 1 et, optionnellement, des excipients ou des véhicules pharmaceutiquement acceptables.

3. Peptide consistant en SEQ ID NO : 1 ou SEQ ID NO : 2 destiné à être utilisé en tant qu'agent thérapeutique.

4. Peptide consistant en SEQ ID NO : 1 ou SEQ ID NO : 2 destiné à être utilisé, selon la revendication 3, dans la prévention ou le traitement de l'ostéoarthrose.

5. Peptide consistant en SEQ ID NO : 1 ou SEQ ID NO : 2 et comprenant en outre une queue N-terminale de 8 arginines, destiné à être utilisé selon les revendications 3 ou 4.

6. Peptide destiné à être utilisé, selon l'une quelconque des revendications 3 à 5, dans lequel le peptide est administré par voie orale ou au moyen d'une injection intraarticulaire, sous-cutanée, intraveineuse ou musculaire.
